# EUROPEAN PATENT SPECIFICATION

(11) **EP 4 206 178 B1**
(45) Date of publication and mention of the grant of the patent: **21.10.2026**
(21) Application number: 22867518.7
(22) Date of filing: 28.06.2022
(51) Int. Cl.: C07C 68/06, B01J 31/02, C07C 69/96

(54) **METHOD FOR PRODUCING ASYMMETRIC LINEAR CARBONATE**
VERFAHREN ZUR HERSTELLUNG VON ASYMMETRISCHEM LINEAREM CARBONAT
PROCÉDÉ DE PRODUCTION DE CARBONATE LINÉAIRE ASYMÉTRIQUE

(30) Priority: 07.09.2021 KR 20210119070
(43) Date of publication of application: 05.07.2023
(73) Proprietor: LG Chem, Ltd., Seoul 07336 (KR)
(72) Inventor: PARK, Jun Beom, Daejeon 34122 (KR); LEE, Hyunyoung, Daejeon 34122 (KR); KIM, Hyun Cheol, Daejeon 34122 (KR)
(74) Representative: Goddar, Heinz J.
(86) International application number: PCT/KR2022/009253
(87) International publication number: WO 2023/038249

(56) References cited:
- EP-B1- 3 950 123
- WO-A1-2021/114091
- WO-A1-2021/114091
- CN-A- 111 138 285
- JP-A- H10 338 663
- LILI YANG: "Zeolitic imidazole framework-67 as an efficient heterogeneous catalyst for the synthesis of ethyl methyl carbonate", vol. 54, 1 September 2014 (2014-09-01), AMSTERDAM, NL, pages 86 - 90, XP093157199, ISSN: 1566-7367, Retrieved from the Internet <URL:https://www.sciencedirect.com/science/article/pii/S1566736714002027/pdfft?md5=4018eae5ffd2485991538c62b8188c95&pid=1-s2.0-S1566736714002027-main.pdf> DOI: 10.1016/j.catcom.2014.05.021
- SONG YU ET AL: "Protic ionic liquid-promoted synthesis of dimethyl carbonate from ethylene carbonate and methanol", CHINESE CHEMICAL LETTERS, ELSEVIER, AMSTERDAM, NL, vol. 31, no. 3, 27 July 2019 (2019-07-27), pages 667 - 672, XP086071482, ISSN: 1001-8417, [retrieved on 20190727], DOI: 10.1016/J.CCLET.2019.07.053
- XIAO ZHU: "DBU-Based Protic Ionic Liquids for CO 2 Capture", ACS SUSTAINABLE CHEMISTRY & ENGINEERING, vol. 5, no. 9, 5 September 2017 (2017-09-05), US, pages 8192 - 8198, XP093156944, ISSN: 2168-0485, DOI: 10.1021/acssuschemeng.7b01839
- JIKUAN QIU: "Efficient Ionic-Liquid-Promoted Chemical Fixation of CO 2 into [alpha]-Alkylidene Cyclic Carbonates", vol. 10, no. 6, 22 March 2017 (2017-03-22), DE, pages 1120 - 1127, XP093157252, ISSN: 1864-5631, Retrieved from the Internet <URL:https://api.wiley.com/onlinelibrary/tdm/v1/articles/10.1002%2Fcssc.201601129> DOI: 10.1002/cssc.201601129
- CHETAN K JADHAV ET AL: "Room temperature ionic liquid promoted improved and rapid synthesis of highly functionalized imidazole and evaluation of their inhibitory activity against human cancer cells", JOURNAL OF THE CHINESE CHEMICAL SOCIETY, CHINESE ELECTRONIC PERIODICAL SERVICES, CHINA, vol. 68, no. 6, 13 January 2021 (2021-01-13), pages 1067 - 1081, XP071028076, ISSN: 0009-4536, DOI: 10.1002/JCCS.202000468
- JIAYIN HU ET AL: "Transformation of Atmospheric CO2 Catalyzed by Protic Ionic Liquids: Efficient Synthesis of 2-Oxazolidinones", ANGEWANDTE CHEMIE INTERNATIONAL EDITION, VERLAG CHEMIE, HOBOKEN, USA, vol. 54, no. 18, 3 March 2015 (2015-03-03), pages 5399 - 5403, XP072081051, ISSN: 1433-7851, DOI: 10.1002/ANIE.201411969
- GU QINGWEN, FANG JIAN, XU ZICHEN, NI WENXIU, KONG KANG, HOU ZHENSHAN: "CO 2 promoted synthesis of unsymmetrical organic carbonate using switchable agents based on DBU and alcohols", NEW JOURNAL OF CHEMISTRY, ROYAL SOCIETY OF CHEMISTRY, GB, vol. 42, no. 15, 1 January 2018 (2018-01-01), GB , pages 13054 - 13064, XP093043503, ISSN: 1144-0546, DOI: 10.1039/C8NJ01638K
- MUNSHI, M ET AL.: "1,8-Diazabicyclo[5.4.0]undec-7-ene (DBU): A highly efficient catalyst in glycerol carbonate synthesis", JOURNAL OF MOLECULAR CATALYSIS A: CHEMICAL, vol. 391, 2014, pages 144 - 149, XP028852034, DOI: 10.1016/j.molcata.2014.04.016

## Description

### [Technical Field]

The present specification relates to a method for preparing an asymmetric linear carbonate.

### [Background Art]

An asymmetric linear carbonate is usually used as an electrolytic solution for a lithium secondary battery, and since the asymmetric linear carbonate has excellent energy storage density, charge capacity, charge/discharge recovery, stability, and the like compared to existing electrolytic solutions, the asymmetric linear carbonate is usually used as an electrolytic solution particularly for a lithium secondary battery.

As a method for preparing an asymmetric linear carbonate, there is a method using an ester reaction of an alkyl chloroformate and an alcohol in the presence of a basic catalyst, but the method has a problem in that the ester reaction is very intense and a highly toxic compound such as phosgene and bisphenol-A needs to be used as a starting material. To complement the problem, there is a method of using a transesterification reaction of a symmetric linear carbonate and an alcohol having an alkyl group in the presence of a basic catalyst such as a metal carbonate, but the method has a problem in that catalytic activity and production yield are low, and an asymmetric linear carbonate such as ethyl methyl carbonate, which is a final target compound needs to be isolated from a total of five reaction products including three types of linear carbonates and two types of alcohols and purified. As another method, a method for preparing an asymmetric linear carbonate in the presence of moisture or alcohol using a mixed oxide of a Group 3 rare earth metal is disclosed, but there is a problem in that the method needs to be performed for a long period of time of 200 hours or more.

WO 2021/114 091 A1 discloses a homogeneous catalyst comprising an ionic liquid with an anion and a cation, wherein the anion and the cation both comprise a nitrogen-containing heterocycle. Furthermore, it is provided a heterogeneous catalyst comprising the homogeneous catalyst, a preparation method and use of the homogeneous catalyst and the heterogeneous catalyst. The homogeneous catalyst and heterogeneous catalyst are used to synthesize high-purity ethyl methyl carbonate product by the transesterification of dimethyl carbonate and ethanol, transesterification of dimethyl carbonate and diethyl carbonate, or transesterification of methanol and diethyl carbonate.

### [Detailed Description of the Invention]

### [Technical Problem]

The present specification has been made in an effort to provide a method for preparing an asymmetric linear carbonate.

### [Technical Solution]

According to an exemplary embodiment of the present specification, provided is a method for preparing an asymmetric linear carbonate, the method including: transesterifying a first symmetric linear carbonate and a second symmetric linear carbonate under a catalyst represented by the following Chemical Formula 1. 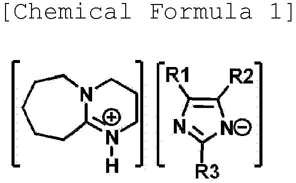 In Chemical Formula 1,
R1 is hydrogen, and R2 and R3 are different from each other, and are each independently hydrogen; a methyl group; or an isopropyl group.

### [Advantageous Effects]

For the method for preparing an asymmetric linear carbonate according to an exemplary embodiment of the present specification, the process is simple and purification is easily performed, so that the asymmetric linear carbonate can be economically prepared.

### [Best Mode]

Hereinafter, the present specification will be described in more detail.

According to the present specification, provided is a method for preparing an asymmetric linear carbonate, the method including: transesterifying a first symmetric linear carbonate and a second symmetric linear carbonate under a catalyst represented by the following Chemical Formula 1. 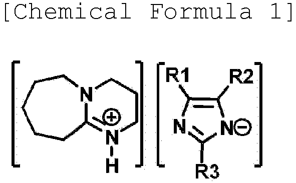 In Chemical Formula 1,
R1 is hydrogen, and R2 and R3 are different from each other, and are each independently hydrogen; a methyl group; or an isopropyl group.

In an exemplary embodiment of the present specification, a portion wherein a substituent is not indicated in Chemical Formula 1 may mean that hydrogen is substituted.

According to an exemplary embodiment of the present specification, the catalyst of Chemical Formula 1 is an ionic liquid catalyst.

According to an exemplary embodiment of the present specification, the catalyst of Chemical Formula 1 is an amidine-based ionic liquid catalyst.

According to an exemplary embodiment of the present specification, the method for preparing an asymmetric linear carbonate does not include a solvent (solvent free).

According to a preparation method in the related art, since two types of linear carbonates and an alcohol-based solvent are used, the preparation process is complicated, and the alcohol-based solvent is used to obtain at least five types of mixtures including a final target compound, so that a purification process for obtaining an asymmetric linear carbonate, which is a final target, is very complicated.

According to an exemplary embodiment of the present specification, since the method for preparing an asymmetric linear carbonate is performed while all of a first symmetric linear carbonate, a second symmetric linear carbonate and the catalyst of Chemical Formula 1, which take part in a transesterification reaction, are liquid materials and homogeneously mixed without using a solvent such as an alcohol-based solvent, a separate solvent is not needed, so that the process is simple, and after the reaction is completed, a first symmetric linear carbonate, a second symmetric linear carbonate and a final target asymmetric linear carbonate, which are a mixture of three types, are only produced, and thus the product is easily isolated and purified, costs for the process are low, and the resulting first symmetric linear carbonate and second symmetric linear carbonate can be used again in the process of preparing the asymmetric linear carbonate, and thus the method for preparing an asymmetric linear carbonate is very economical compared to the method in the related art.

According to an exemplary embodiment of the specification, the mixture of three types may be isolated and purified through fractional distillation using a difference in boiling points.

According to an exemplary embodiment of the present specification, the first symmetric linear carbonate is dimethyl carbonate (DMC).

According to an exemplary embodiment of the present specification, the second symmetric linear carbonate is diethyl carbonate (DEC).

According to an exemplary embodiment of the present specification, the temperature of the transesterification reaction is 80°C to 120°C, preferably 90°C to 100°C, and more preferably 95°C to 100°C.

When the reaction is performed at the reaction temperature, the first symmetric linear carbonate and the transesterification rate of the second symmetric linear carbonate are optimized and the yield of the asymmetric linear carbonate is excellent.

According to an exemplary embodiment of the present specification, the transesterification reaction is a closed system reaction. The closed system reaction is a physical system that exchanges only energy without exchanging materials with the outside, and the transesterification reaction is performed using only the first symmetric linear carbonate, the second symmetric linear carbonate and the catalyst represented by Chemical Formula 1 without the inflow of external materials.

In the present specification, the alkyl group is straight-chained or branched, and the number of carbon atoms of the alkyl group is 1 to 4. Specific examples of the alkyl group may be a methyl group, an ethyl group, an n-propyl group, an isopropyl group, an n-butyl group, an isobutyl group, a tert-butyl group and the like.

According to an exemplary embodiment of the present specification, the catalyst represented by Chemical Formula 1 is any one selected among the following compounds.

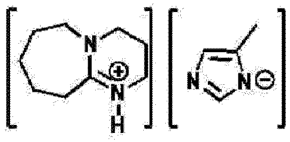

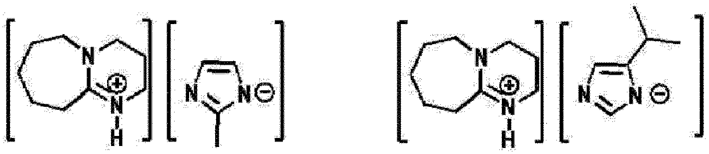

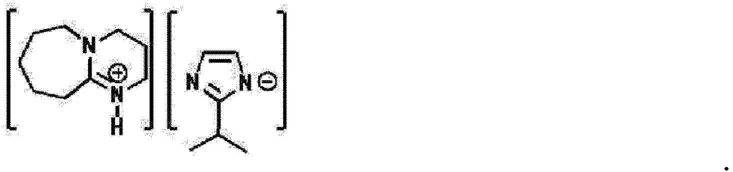

According to an exemplary embodiment of the present specification, the asymmetric linear carbonate prepared by the method for preparing an asymmetric linear carbonate is ethyl methyl carbonate (EMC).

According to an exemplary embodiment of the present specification, the asymmetric linear carbonate is ethyl methyl carbonate (EMC).

According to an exemplary embodiment of the present specification, the asymmetric linear carbonate may be used as an electrolytic solution for a lithium secondary battery.

Hereinafter, the present specification will be described in detail with reference to Examples for specifically describing the present specification.

### Mode for invention

The (Reference) Examples of the present specification are provided to more clearly describe the present specification to a person with ordinary skill in the art.

### Synthesis Example 1. Preparation of Reference Compound 1

0.6 g of imidazole and 1.5 g of 1,8-diazabicyclo(5.4.0)undec-7-ene were put into a 20-ml vial, and the resulting mixture was stirred at room temperature for 5 hours. When the reaction was completed, Compound 1, which is a pale yellow liquid, was obtained.

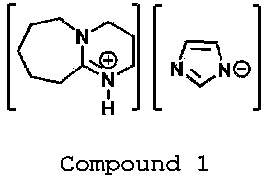

### Synthesis Example 2. Preparation of Compound 2

Compound 2 was obtained by synthesis in the same manner as in Synthesis Example 1, except that 0.8 g of 2-methyl-1H-imidazole was used instead of imidazole.

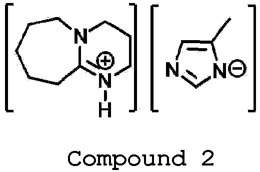

### Synthesis Example 3. Preparation of Compound 3

Compound 3 was obtained by synthesis in the same manner as in Synthesis Example 1, except that 0.8 g of 4-methyl-1H-imidazole was used instead of imidazole.

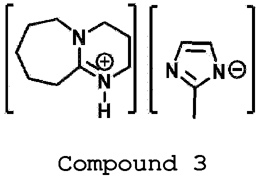

### Synthesis Example 4. Preparation of Compound 4

Compound 4 was obtained by synthesis in the same manner as in Synthesis Example 1, except that 1.1 g of 2-isopropyl-1H-imidazole was used instead of imidazole.

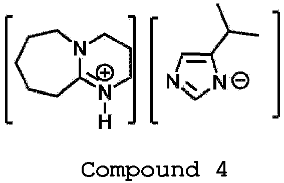

### Synthesis Example 5. Preparation of Compound 5

Compound 5 was obtained by synthesis in the same manner as in Synthesis Example 1, except that 1.1 g of 4-isopropyl-1H-imidazole was used instead of imidazole.

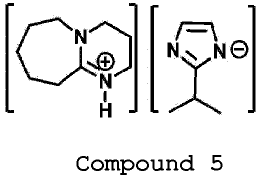

### Experimental Examples. Preparation of ethyl methyl carbonate

### Reference Example 1

10 g of dimethyl carbonate (DMC), 13.1 g of diethyl carbonate (DEC) (DMC:DEC = 1:1, molar ratio) and 1 wt% of Reference Compound 1 (DMC standard 1 wt%) as a catalyst were put into a 100-mL pressure reactor. The temperature was increased to 90°C to 100°C, and the reaction was performed under stirring for 4 hours. When the reaction was completed, a gas chromatography (GC) analysis was performed by performing sampling.

The reaction was performed in a closed system, and additional pressure was not applied.

The reaction is performed as shown in the following Reaction Scheme 1, and no product is produced other than the three types of materials.

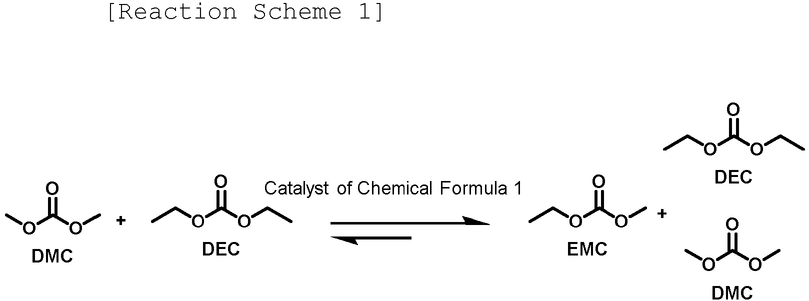

### Example 2

The reaction was performed in the same manner as in Reference Example 1, except that as the catalyst, Compound 2 was used instead of Compound 1.

### Example 3

The reaction was performed in the same manner as in Reference Example 1, except that as the catalyst, Compound 3 was used instead of Compound 1.

### Example 4

The reaction was performed in the same manner as in Reference Example 1, except that as the catalyst, Compound 4 was used instead of Compound 1.

### Example 5

The reaction was performed in the same manner as in Reference Example 1, except that as the catalyst, Compound 5 was used instead of Compound 1.

### Comparative Example 1

Dimethyl carbonate (DMC) (10 g), ethanol (EtOH) (5.1 g) (DMC:EtOH = 1:1, molar ratio) and 1 wt% (DMC standard 1 wt%) of sodium ethoxide as a catalyst were put into a 100-mL pressure reactor. The temperature was increased to 90°C to 100°C, and the reaction was performed under stirring for 4 hours. When the reaction was completed, a GC analysis was performed by performing sampling.

The results of GC analysis of the materials prepared by the preparation methods of Examples 1 to 5 and Comparative Example 1 are shown in the following Table 1.

**[Table 1]**

| | Before reaction (mole%) | | After reaction (mole%) | | | | | | |
|---|---|---|---|---|---|---|---|---|---|
| | DMC | DEC | DMC | | EMC | | | DEC | |
| Reference Example 1 | 50 | 50 | 23.1 | | 51.2 | | | 25.7 | |
| Example 2 | | | 23.0 | | 50.9 | | | 26.1 | |
| Example 3 | | | 24.0 | | 50.5 | | | 25.5 | |
| Example 4 | | | 23.7 | | 50.8 | | | 25.5 | |
| Example 5 | | | 22.8 | | 51.1 | | | 26.1 | |
| | DMC | EtOH | DMC | EMC | | DEC | MeOH | | EtOH |
| Comparative Example 1 | 50 | 50 | 22.4 | 22.8 | | 5.7 | 38.9 | | 10.1 |

| | | | | | | | | | |
|---|---|---|---|---|---|---|---|---|---|
| - DMC: Dimethyl carbonate - DEC: Diethyl carbonate - EMC: Ethylmethyl carbonate - MeOH: Methanol - EtOH: Ethanol | | | | | | | | | |

In Table 1, in Comparative Example 1 using an alcohol-based solvent which is a method in the related art, a mixture of 5 types including EMC which is a final target is produced, so that it is difficult to isolate and purify the EMC.

In contrast, since the preparation method according to the present specification is performed while all of a first symmetric linear carbonate, a second symmetric linear carbonate and the catalyst of Chemical Formula 1, which take part in a transesterification reaction, are liquid materials and homogeneously mixed without using a solvent such as an alcohol-based solvent, a separate solvent is not needed, so that the process is simple. Furthermore, after the reaction was completed, DMC, DEC and a final target EMC, which are a mixture of three types, were only produced, the EMC is easily isolated and purified, costs for the process are low, and DMC and DEC can also be used again for the process of preparing an asymmetric linear carbonate, so that it could be seen that the preparation method according to an exemplary embodiment of the present specification is very economical compared to methods in the related art.

## Claims

1. A method for preparing an asymmetric linear carbonate, the method comprising: transesterifying a first symmetric linear carbonate and a second symmetric linear carbonate under a catalyst represented by the following Chemical Formula 1: 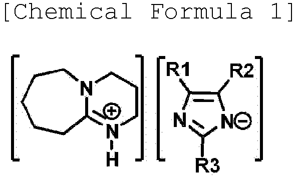 wherein, in Chemical Formula 1,
R1 is hydrogen, and R2 and R3 are different from each other, and are each independently hydrogen; a methyl group; or an isopropyl group.

2. The method for preparing an asymmetric linear carbonate of claim 1, wherein the method does not comprise a solvent (solvent free).

3. The method for preparing an asymmetric linear carbonate of claim 1, wherein the first symmetric linear carbonate is dimethyl carbonate (DMC).

4. The method for preparing an asymmetric linear carbonate of claim 1, wherein the second symmetric linear carbonate is diethyl carbonate (DEC).

5. The method for preparing an asymmetric linear carbonate of claim 1, wherein a temperature of the transesterification reaction is 80°C to 120°C.

6. The method for preparing an asymmetric linear carbonate of claim 1, wherein the catalyst represented by Chemical Formula 1 is any one selected among the following compounds:

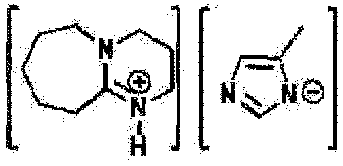

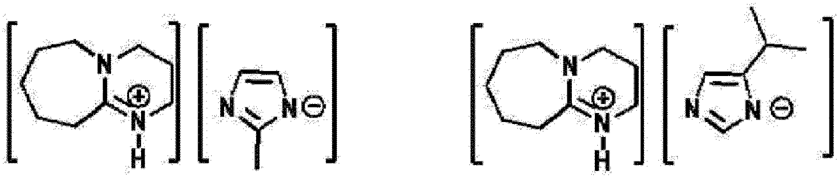

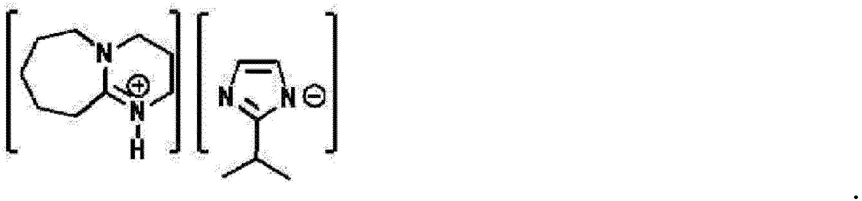

7. The method for preparing an asymmetric linear carbonate of claim 1, wherein the asymmetric linear carbonate is ethyl methyl carbonate (EMC).

## Patentansprüche

1. Verfahren zur Herstellung eines asymmetrischen linearen Carbonats, wobei das Verfahren umfasst: Umestern eines ersten symmetrischen linearen Carbonats und eines zweiten symmetrischen linearen Carbonats unter einem Katalysator, der durch die folgende chemische Formel 1 dargestellt wird: 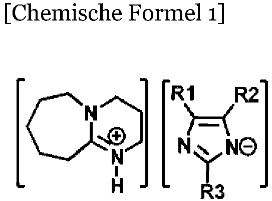 wobei in der chemischen Formel 1
R1 Wasserstoff ist und R2 und R3 voneinander verschieden sind und jeweils unabhängig Wasserstoff; eine Methylgruppe; oder eine Isopropylgruppe sind.

2. Verfahren zur Herstellung eines asymmetrischen linearen Carbonats nach Anspruch 1, bei dem das Verfahren kein Lösungsmittel (lösungsmittelfrei) umfasst.

3. Verfahren zur Herstellung eines asymmetrischen linearen Carbonats nach Anspruch 1, bei dem das erste symmetrische lineare Carbonat Dimethylcarbonat (DMC) ist.

4. Verfahren zur Herstellung eines asymmetrischen linearen Carbonats nach Anspruch 1, bei dem das zweite symmetrische lineare Carbonat Diethylcarbonat (DEC) ist.

5. Verfahren zur Herstellung eines asymmetrischen linearen Carbonats nach Anspruch 1, bei dem eine Temperatur der Umesterungsreaktion 80 °C bis 120 °C beträgt.

6. Verfahren zur Herstellung eines asymmetrischen linearen Carbonats nach Anspruch 1, bei dem der durch die chemische Formel 1 dargestellte Katalysator einer ist, der aus den folgenden Verbindungen ausgewählt ist: 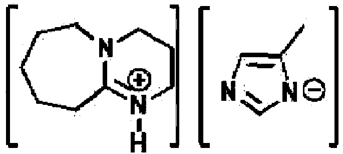 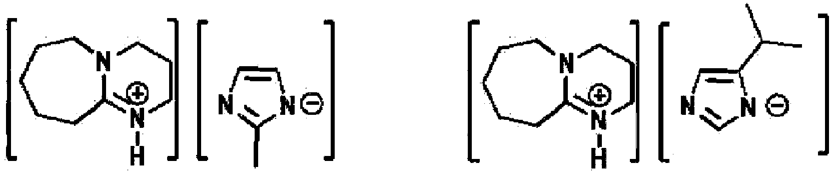 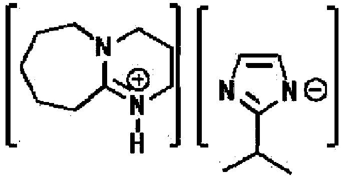 .

7. Verfahren zur Herstellung eines asymmetrischen linearen Carbonats nach Anspruch 1, bei dem das asymmetrische lineare Carbonat Ethylmethylcarbonat (EMC) ist.

## Revendications

1. Procédé de préparation d'un carbonate linéaire asymétrique, le procédé consistant à : transestérifier un premier carbonate linéaire symétrique et un deuxième carbonate linéaire symétrique sous un catalyseur représenté par la Formule chimique 1 ci-après : 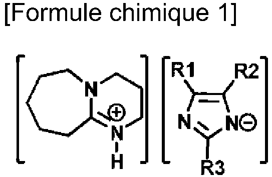 dans lequel, dans la Formule chimique 1,
R1 est un hydrogène, et R2 et R3 sont différents l'un de l'autre, et sont chacun indépendamment un hydrogène ; un groupe méthyle ; ou un groupe isopropyle.

2. Procédé de préparation d'un carbonate linéaire asymétrique selon la revendication 1, dans lequel le procédé ne comprend pas de solvant (sans solvant).

3. Procédé de préparation d'un carbonate linéaire asymétrique selon la revendication 1, dans lequel le premier carbonate linéaire symétrique est un carbonate de diméthyle (DMC).

4. Procédé de préparation d'un carbonate linéaire asymétrique selon la revendication 1, dans lequel le deuxième carbonate linéaire symétrique est un carbonate de diéthyle (DEC).

5. Procédé de préparation d'un carbonate linéaire asymétrique selon la revendication 1, dans lequel une température de la réaction de transestérification est 80°C à 120°C.

6. Procédé de préparation d'un carbonate linéaire asymétrique selon la revendication 1, dans lequel le catalyseur représenté par la Formule chimique 1 est un ou plusieurs éléments sélectionnés parmi les composés suivants :

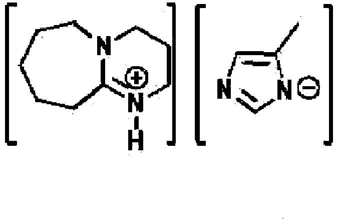

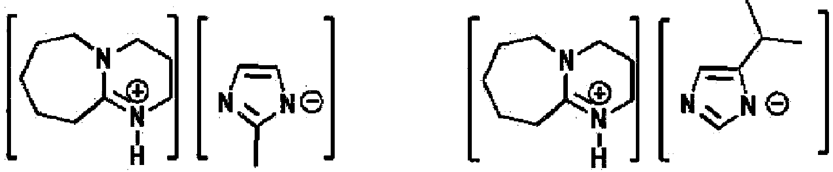

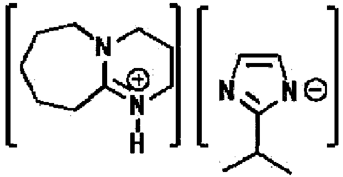

7. Procédé de préparation d'un carbonate linéaire asymétrique selon la revendication 1, dans lequel le carbonate linéaire asymétrique est un carbonate d'éthylméthyle (EMC).
